# EUROPEAN PATENT SPECIFICATION

(11) **EP 1 208 097 B1**
(45) Date of publication and mention of the grant of the patent: **18.02.2009**
(21) Application number: 00951781.4
(22) Date of filing: 26.07.2000
(51) Int. Cl.: C07D 403/06, C07D 401/06, C07D 409/14, C07D 409/12, C07D 471/04, C07D 241/08, C07D 495/04, C07D 401/14, C07D 409/06, C07D 403/14, C07D 491/04, C07D 417/14, A61K 31/496, A61P 7/02

(54) **SUBSTITUTED OXOAZAHETEROCYCLYL COMPOUNDS**
SUBSTITUIERTE OXOAZAHETEROZYCLISCHE VERBINDUNGEN
COMPOSES D'OXOAZAHETEROCYCLYLE SUBSTITUE

(30) Priority: 28.07.1999 US 363196
(43) Date of publication of application: 29.05.2002
(73) Proprietor: Aventis Pharmaceuticals Inc., Bridgewater, New Jersey 08807 (US)
(72) Inventor: EWING, William, R., Downingtown, PA 19335 (US); BECKER, Michael, R., Norristown, PA 19401 (US); CHOI-SLEDESKI, Yong, Mi, Collegeville, PA 19426 (US); PAULS, Heinz, W., Collegeville, PA 19426 (US); HE, Wei, Collegeville, PA 19426 (US); CONDON, Stephen, M., Chester Springs, PA 19425 (US); DAVIS, Roderick, S., West Chester, PA 19382 (US); HANNEY, Barbara, A., Pennsburg, PA 18073 (US); SPADA, Alfred, P., Lansdale, PA 19446 (US); BURNS, Christopher J., Rosemont, PA 19010 (US); JIANG, John, Z., Collegeville, PA 19426 (US); LI, Aiwen, Audubon, PA 19403 (US); MYERS, Michael, R., Reading, PA 19606 (US); LAU, Wan, F., Groton, CT 06340-5627 (US); POLI, Gregory, B., Perkasie, PA 18944 (US)
(74) Representative: Then, Johann
(86) International application number: PCT/IB2000/001156
(87) International publication number: WO 2001/007436

(56) References cited:
- WO-A-00/32590
- WO-A-96/40679
- WO-A-98/21188
- WO-A-98/46591
- WO-A-99/33805
- WO-A-99/37304
- WO-A-99/40075

## Description

### FIELD OF THE INVENTION

This invention is directed to oxoazaheterocyclyl compounds which inhibit Factor

This invention is directed to oxoazaheterocyclyl compounds which inhibit Factor Xa, to pharmaceutical compositions comprising these compounds, to intermediates useful for preparing these compounds and to a method of inhibiting Factor Xa. This invention is also directed to oxoazaheterocyclyl compounds which directly inhibit both Factor Xa and Factor IIa (thrombin), to pharmaceutical compositions comprising these compounds, to intermediates useful for preparing these compounds and to a method of simultaneously directly inhibiting both Factor Xa and Factor IIa (thrombin).

### BACKGROUND OF THE INVENTION

Factor Xa and Factor Xa assembled in the prothrombinase complex (Factor Xa, Factor Va, calcium and phospholipid) activate prothrombin (Factor II) to generate thrombin (Factor IIa). Factor Xa is strategically located at the intersection of extrinsic and intrinsic pathways of the blood coagulation system. Thus, an inhibitor of Factor Xa inhibits the formation of thrombin and, therefore, is useful for preventing or treating disorders related to blood coagulation in mammals.

Anticoagulant therapy is indicated for the treatment and prophylaxis of a variety of thrombotic conditions of both the venous and arterial vasculature. In the arterial system, abnormal thrombus formation is primarily associated with arteries of the coronary, cerebral and peripheral vasculature. The diseases associated with thrombotic occlusion of these vessels principally include acute myocardial infarction (AMI), unstable angina, thromboembolism, acute vessel closure associated with thrombolytic therapy and percutaneous transluminal coronary angioplasty (PTCA), transient ischemic attacks, stroke, intermittent claudication and bypass grafting (CABG) of the coronary or peripheral arteries. Chronic anticoagulant therapy may also be beneficial in preventing the vessel luminal narrowing (restenosis) that often occurs following PTCA and CABG, and in the maintenance of vascular access patency in long-term hemodialysis patients. With respect to the venous vasculature, pathologic thrombus formation frequently occurs in the veins of the lower extremities following abdominal, knee and hip surgery (deep vein thrombosis, DVT). DVT further predisposes the patient to a higher risk of pulmonary thromboembolism. A systemic, disseminated intravascular coagulopathy (DIC) commonly occurs in both vascular systems during septic shock, certain viral infections and rapid consumption of coagulation factors and their plasma inhibitors resulting in the formation of life-threatening clots throughout the microvasculature of several organ systems.

In addition to their use in anticoagulant therapy, Factor Xa inhibitors are useful in the treatment or prevention of other diseases in which the generation of thrombin has been implicated as playing a physiologic role. For example, thrombin has been proposed two contribute to the morbidity and mortality of such chronic and degenerative diseases as arthritis, cancer, atherosclerosis and Alzheimer's disease by virtue of its ability to regulate many different cell types through specific cleavage and activation of a cell surface thrombin receptor, mitogenic effects, diverse cellular functions such as cell proliferation, for example, abnormal proliferation of vascular cells resulting in restenosis or angiogenesis, release of PDGF and DNA syntheses. Inhibition of Factor Xa will effectively block thrombin generation and therefore neutralize any physiologic effects of thrombin on various cell types.

The representative indications discussed above include some, but not all, of the possible clinical situations amenable to treatment with a Factor Xa inhibitor.

Oxoazaheterocyclyl Factor Xa inhibitors are disclosed in International Patent Application Numbers PCT/US98/07158, published Oct. 22, 1998; PCT/US98/07159, published Oct. 22, 1998; PCT/US98/07160, published Oct. 22, 1998; PCT/US98/07161, published Oct. 22, 1998; and PCT/US96/09290, published Dec. 19, 1996. Oxoazaheterocyclyl fibrinogen antagonists are disclosed in International Patent Application Number PCT/US92/09467, published May 13, 1993.
Substituted oxoazaheterocyclyl Factor Xa inhibitors are disclosed in International Patent Application Number WO 99/37304.

Vascular injury, caused by biochemical or physical perturbations, results in the activation of the coagulation system, culminating in the generation of thrombin. Thrombin promotes thrombus formation by catalyzing the transformation of fibrinogen to fibrin, by activating Coagulation Factor XIII, which stabilizes the thrombus, and by activating platelets. Thrombin promotes further thrombus growth by positive feedback to the coagulation cascade (activation of Coagulation Factors V and VIII), resulting in the explosive production of thrombin. Thrombin is present, and active, in the thrombi of patients with thrombotic vascular disease. Thrombin inhibition prevents the action of thrombin after thrombin has been activated from prothrombin. An inhibitor of thrombin inhibits cleavage of fibrinogen to fibrin, activation of Factor Xllla, activation of platelets, and feedback of thrombin to the coagulation cascade to generate more thrombin. Consequently, inhibition of thrombin activity with a direct thrombin inhibitor would be useful for preventing or treating disorders related to blood coagulation in mammals.

The combined inhibitors of Factor Xa and Factor IIa described herein inhibit thrombin *activity* (via IIa inhibition) and thrombin *production* (via Factor Xa inhibition). Therefore, these agents inhibit any thrombin that may be present and also inhibit the further production of thrombin. Other agents which have this dual activity include heparin and low molecular weight heparins (LMWHs), which have demonstrated efficacy in thrombotic diseases However, heparin and LMWHs act indirectly through a cofactor, antithrombin-III (ATIII), to inhibit Xa and IIa. The heparin/ATIII complex is too large, however, to inhibit thrombus-bound Xa and IIa, thus limiting its efficacy. Direct inhibitors of Factor Xa and Factor IIa, as described herein, are capable of inhibiting soluble and thrombus-bound Xa and IIa, thus providing an important therapeutic advantage over currently available Xa/IIa inhibitors.

### SUMMARY OF THE INVENTION

This invention is directed to a compound selected from the group consisting of 1-(4-Amino-quinazolin-7-ylmethyl)-4-[5-(5-chloro-thiophen-2-yl)-isoxazol-3-ylmethyl]-piperazine-2,3-dione,
(*R*)-1-(4-Amino-quinazolin-7-ylmethyl)-4-[5-(5-chloro-thiophen-2-yl)-isoxazol-3-ylmethyl]-6-methoxymethyl-piperazin-2-one,
(6R)-1-(4-Amino-quinazolin-7-ylmethyl)-4-[5-(5-chloro-thiophen-2-yl)-isoxazol-3-ylmethyl]-6-ethoxymethyl-piperazin-2-one,
1-(4-Amino-quinazolin-7-ylmethyl)-4-[5-(5-chloro-thiophen-2-yl)-isoxazol-3-ylmethyl]-3-(S)-propyl-piperazin-2-one ditrifluoroacetate,
1-(4-Amino-quinazolin-7-ylmethyl)-4-[5-(5-chlore-thiophen-2-yl)-isoxazol-3-ylmethyl]-3-(S)-methyl-piperazin-2-one ditrifluoroacetate,
1-(4-Amino-quinazolin-7-ylmethyl)-4-[3-(5-chloro-thiophen-2-yl)-isoxazol-5-ylmethyl]-piperazin-2-one ditrifluoroacetate,
1-(4-Amino-quinazolin-7-ylmethyl)-4-[3-(5-chloro-thiophen-2-yl)-isoxazol-5-ylmethyl]-3-methyl-piperazin-2-one ditrifluoroacetate,
1-(4-Amino-quinazolin-7-ylmethyl)-4-[5-(5-chloro-thiophen-2-yl)-isoxazol-3-ylmethyl]-piperazin-2-one ditrifluoroacetate or
4-[5-(5-Chloro-thiophen-2-yl)-isoxazol-3-ylmethyl]-1-(1H-pyrrolo[3,2-c]pyridin-2-ylmethyl)-piperazin-2-one ditrifluoroacetate.

### DETAILED DESCRIPTION OF THE INVENTION

### Preferred Embodiments

A preferred aspect of the invention is 1-(4-Amino-quinazolin-7-ylmethyl)-4-[5-(5-chloro-thiophen-2-yl)₋isoxazol-3-ylmethyl]-piperazine-2,3-dione,
(*R*)-1-(4-Amino-quinazolin-7-ylmethyl)-4-[5-(5-chloro-thiophen-2-yl)-isoxazol-3-ylmethyl]-6-methoxymethyl-piperazin-2-one,
(6R)-1-(4-Amino-quinazolin-7-ylmethyl)-4-[5-(5-chloro-thiophen-2-yl)-isoxazol-3-ylmethyl]-6-ethoxymethyl-piperazin-2-one,
1-(4-Amino-quinazolin-7-ylmethyl)-4-[5-(5-chloro-thiophen-2-yl)-isoxazol-3-ylmethyl]-3-(S)-propyl-piperazin-2-one ditrifluoroacetate,
1-(4-Amino-quinazolin-7-ylmethyl)-4-[5-(5-chloro-thiophen-2-yl)-isoxazol-3-ylmethyl]-3-(S)-methyl-piperazin-2-one ditrifluoroacetate,
1-(4-Amino-quinazolin-7-ylmethyl)-4-[3-(5-chloro-thiophen-2-yl)-isoxazol-5-ylmethyl]-piperazin-2-one ditrifluoroacetate,
1-(4-Amino-quinazolin-7-ylmethyl)-4-[3-(5-chloro-thiophen-2-yl)-isoxazol-5-ylmethyl]-3-methyl-piperazin-2-one ditrifluoroacetate,
1-(4-Amino-quinazolin-7-ylmethyl)-4-[5-(5-chloro-thiophen-2-yl)-isoxazol-3-ylmethyl]-piperazin-2-one ditrifluoroacetate or
4-[5-(5-Chloro-thiophen-2-yl)-isoxazol-3-ylmethyl]-1-(H-pyrrolo[3,2-c]pyridin-2-ylmethyl)-piperazin-2-one ditrifluoroacetate.

### Preparation of the Compounds of the Invention

This invention is further exemplified by the following examples which further illustrate the preparation of the compounds of this invention. The starting materials and intermediates are prepared by the application or adaptation of known methods, for example methods used heretofore or described in the literature, for example those described by R. C. Larock in Comprehensive Organic Transformations, VCH publishers, 1989.

### Reference EXAMPLE 72. 1-(4-Aminoquinazoline-7-ylmethyl)piperazine-2-one.

### A. 4-(4-Chloroquinazoline-7-ylmethyl)-3-oxopiperazine-1-carboxylic acid tert-butyl ester.

To a solution of 3-oxopiperazine-1-carboxylic acid tert-butyl ester (3.93 g, 19.6 mmol) and 7-bromomethyl-4-chloroquinazoline, EXAMPLE 7, (5.0 g, 19.6 mmol) in 150 mL of THF and 15 mL of DMF at 0°C is added a 60% dispersion in mineral oil of NaH (0.79 g, 19.6 mmol). The solution is stirred at 0°C for 0.5 hours and then is allowed to warm to ambient temperature. After 4 hours, the solution is poured into a saturated solution of NH₄Cl. The layers are separated and the organic layer is washed with H₂O, and saturated NaCl, dried over MgSO₄, filtered and concentrated. The title compound is obtained as a white solid (5.1 g, 13.4 mmol). MS (FAB) m/z 377, 379, (M+H), chlorine pattern.

### B. 4-(4-Aminoquinazoline-7-ylmethyl-3-oxopiperazine-1-carboxylic acid tert-butyl ester.

A solution of 4-(4-chloroquinazoline-7-ylmethyl)-3-oxopiperazine-1-carboxylic acid tert-butyl ester (1.84 g, 4.9 mmol) in 120 mL of ethanol is saturated with NH₃ gas. To the resulting solution is added acetic acid (0.03 mL). The solution is heated to reflux. After 16 hours, the solution is concentrated. The resulting solid is dissolved in CH₂Cl₂ and the inorganic salts are filtered off. The organic solution is concentrated. The resulting solid is triturated with EtOAc. The title compound is obtained a a white solid (1.59 g, 4.5 mmol). MS (FAB) m/z 356, (M+H).

### C. 1-(4-Aminoquinazoline-7-ylmethyl)piperazine-2-one.

A solution of 4-(4-aminoquinazoline-7-ylmethyl)-3-oxo-piperazine-1-carboxylic acid tert-butyl ester (1.92 g, 5.4 mmol) in EtOAc (200 mL) at 0 °C is saturated with HCl gas. The solution is stirred at 0°C for 4 hours. After this time, the solution is concentrated. The title compound is obtained as a white solid (1.79 g, 5.4 mmol). ¹HNMR (d⁶-DMSO, 300 MHz) δ 9.9 (bs, 3H), 9.7 (bs, 2H), 8.8 (s, 1H), 8.46 (d, 1 H), 7.72 (s, 1H), 7.61 (d, 1 H), 4.78 (s, 2H), 3.83 (s, 2H), 3.4 (m, 4H).

### Reference EXAMPLE 101. 4-[4-(6-Chlorobenzo[b]thiophene-2-sulfonyl)-2-oxo-piperazine-1-ylmethyl]benzamidine.

To a solution of 4-(2-oxopiperazin-1-ylmethyl)benzamidine bistrifluoroacetate (0.38 g, 0.83mmol), EXAMPLE 66, in CH₂Cl₂ (5 mL) is added Et₃N (0.35 mL, 2.6 mmol) and 6-chlorobenzo[b]thiophene-2-sulfonyl chloride (0.23 g, 0.85 mmol, EXAMPLE 1. After 6 hours, the solution is concentrated. The product is purified by RP-HPLC eluting in a gradient of 10% CH₃CN/H₂O(0.1% TFA) to 70% CH₃CN/H₂O(0.1 % TFA). The appropriate collected fractions are lyophilized to afford the title compound as a white solid (0.37 g, 0.65 mmol). ¹H NMR (d⁶-DMSO, 300MHz) δ 9.33 (bs, 2H), 8.96 (bs, 2H), 8.30 (s, 1H), 8.18 (s, 1H), 8.04 (d, 1H), 7.70 (m, 2H), 7.50 (m, 1H), 7.28 (m, 2H), 4.55 (s, 2H), 3.86 (s, 2H), 3.44 (m, 2H), 3.22 (m, 2H).

The following compound is prepared from the compound of Example 72 and the appropriate sulfonyl choride using the method of Example 101.

| Reference | Name | m/z (M+H) |
|---|---|---|
| Example | | |
| 190 | 1-(4-Amino-quinazolin-7-ylmethyl)-4-(5-isoxazol-3-yl-thiophene-2-sulfonyl)-piperazin-2-one | 471 |

### Reference EXAMPLE 1307 4-[(5-Chlorothiophen-2-yloxy)-acetyl]-l-[(2-([N,N-dimethylaminoethyl]-amino)-pyrimidin-5-yl)-methyl]-3-(S)-methoxymethyl-piperazine-2-one

### A. 5-hydroxymethyl-2-methylthiopyrimidine

To a solution of 2-methylthiopyrimidine-5-carboxaldehyde (1.35 g, 8.7 mmol), prepared according to the method of Gupton et al. (*J. Het. Chem.* 28, 1991, 1281), in methanol (1 mL) at 0°C is added sodium borohydride (0.3 g, 7.9 mmol). The mixture is stirred for 0.5 h and is concentrated *in vacuo.* The residue is partitioned between EtOAc and 1 N NaOH. The organic phase is dried (MgSO₄) and is evaporated to yield the intermediate title compound as a yellow solid (1.18 g, 87%). MS (ES), 157 [M+H]⁺.

### B. 5-bromomethyl-2-methylthiopyrimidine

5-hydroxymethyl-2-methylthiopyrimidine (0.1 g, 0.61 mmol), triphenylphosphine (0.45 g, 1.7 mmol) and carbon tetrabromide (0.28 g, 0.85 mmol) are stirred in benzene (5 mL) for 24 h. The mixture is evaporated and the residue is purified by flash chromatography (silica gel, 4:1 1 hexanes/ethyl acetate) to provide the intermediate title compound as a white solid (0.08 g, 61%). MS (ES), 219/221 [M+H]⁺ (Br).

### C. 4-benzyloxycarbonyl-3-(S)-methoxymethyl-1-[(2-methylthiopyrimidin-5-yl)-methyl]-piperazine-2-one

4-Benzyloxycarbonyl-3-(*S*)-methoxymethyl-piperazine-2-one (0.1 g 0.37 mmol), 5-bromomethyl-2-methylthiopyrimidine (0.08 g, 0.37 mmol) and tetra-n-butylammonium bromide (0.06 g, 0.19 mmol) are placed in dichloromethane (1 mL) and 50% aqueous NaOH (0.03 mL) and stirred for 4 h. The mixture is diluted with water and is extracted with dichloromethane (2 X 20 mL). The combined organic extracts are dried (MgSO₄) and are evaporated. The residue is purified by flash chromatography (silica gel, 98:2 dichloromethane/methanol) to provide the intermediate title compound as a colorless oil (0.05 g, 33%). MS (ES), 417 [M+H]⁺.

### D. 4-benzyloxycarbonyl-1-[(2-{[N,N-dimethylaminoethyl]-amino}pyrimidin-5-yl)-methyl]-3-(S)-methoxymethyl-piperazine-2-one

4-benzyloxycarbonyl-3-(S)-methoxymethyl-1-[(2-methylthiopyrimidin-5-yl)-methyl]-piperazine-2-one (0.045 g, 0.11 mmol) is dissolved in dichloromethane (3 mL) and cooled to -78°C. 57-86% 3-Chloroperoxybenzoic acid (0.095 g, 0.33 mmol) is added and the mixture is warmed to room temperature. The mixture is diluted with dichloromethane (20 mL) and is washed with dilute aqueous Na₂CO₃. The organic phase is dried (Na₂SO₄) and is evaporated. The crude residue is used without further purification. MS (ES), 449 [M+H]⁺. The residue is placed in DMF (1 mL) and N,N-dimethylethylamine (0.05 g, 0.6 mmol) is added. The mixture is stirred for 4 h and is concentrated *in vacuo.* Purification by flash chromatography (silica gel, 9:1 dichloromethane/methanol) provided the intermediate title compound as a colorless resin (0.01 g, 20%). MS (ES), 457 [M+H]⁺.

### E. 1-[(2-{[N,N-dimethylaminoethyl]-amino}-pyrimidin-5-yl)-methyl]-3-(S)-methoxymethyl-piperazine-2-one

4-Benzyloxycarbonyl-1-[(2-{[N, N-dimethylaminoethyl]-amino}-pyrimidin-5-yl)-methyl]-3-(*S*)-methoxymethyl-piperazine-2-one (0.01 g, 0.02 mmol) and 10% Pd on carbon (0.01 g) are stirred in acetic acid (3 mL) under a hydrogen atmosphere for 18 h. The mixture is filtered through Celite^{@} and is evaporated to provide the intermediate title compound as a clear colorless oil (0.002 g). MS (ES), 323 [M+H]⁺.

### F. 4-[(5-Chlorothiophen-2-yloxy)-acetyl]-1-[(2-{[N,N-dimethylaminoethyl]-amino}-pyrimidin-5-yl)-methyl]-3-(S)-methoxymethyl-piperazine-2-one

The title compound can be prepared by placing 1-[(2-{[N,N-dimethylaminoethyl]-amino}-pyrimidin-5-yl)-methyl]-3-(*S*)-methoxymethyl-piperazine-2-one, (5-chloro-thiophen-2-yloxy)-acetic acid, 2-(1H-benzotriazol-1-yl)-1,1,3,3-tetramethyluronium tetrafluoroborate and triethylamine in DMF and stirring 8-16 h. The mixture is evaporated and is diluted with ethyl acetate. The organic phase is washed with water, 2 N HCl, 1 N NaOH and brine, is dried (MgSO₄) and is evaporated. The residue is purified by flash chromatography (silica gel, 4:1 ethyl acetate/hexanes) to provide the title compound.

### Reference EXAMPLE 1308. 1-(4-Amino-quinazolin-7-ylmethyl)-4-[3-(4-chloro-phenyl)-allyl]-piperazine-2,3-dione.

### A. Methyl 2-amino -4-hydroxymethlybenzoate.

To a solution of 16.0 g (76.6 mmole) of dimethyl aminoterephthalate in 200 ml of anhydrous THF cooled to -78°C is added 250 ml (250 mmole) of 1 M Super Hydride dropwise over 1 hour. The mixture is stirred for an additional 1.5 hours warming to 0°C (a little starting material on TLC is observed). The mixture is poured into 300 ml of cold water and extracted with ethyl acetate. The organic layer is washed with water and the two layers are allowed to stand for 30 minutes. The organic layer is dried over MgSO₄ and filtered. The filtrate is evaporated. The residue is dissolved in ethyl acetate and the solution is poured over a Buchner funnel containing silica gel, using 150 ml of ethyl acetate to wash the funnel. The filtrate is evaporated. The residue is dissolved in the minimum amount of ethyl acetate and the solution is diluted to the cloudy point with hexane. Additional hexane is added as the product precipitates. A total of 100 ml of hexane is added and the solid is collected and vacuum dried to give 8.4 g of the title intermediate material, 98-100°C mp; 61% yield. ¹H NMR (CDCl₃, 300MHz) δ 7.82 (d, 1H), 6.67 (s, 1H), 6.60 (d, 1 H), 5.75 (bs, 2H), 4.62 (s, 2H), 3.86 (s, 3H), 1.83 (bs, 1 H). EI MS, [M]⁺=181.

### B. 7-Hydroxymethlyquinazolin-4-one.

A mixture of 2.0 g (19.1 mmole) of methyl 2-amino-4-hydroxymethlybenzoate in 4 ml of formamide is heated in an oil bath of 180°C for three hours. The mixture is cooled and triturated with 70 ml of boiling ethyl acetate. The ethyl acetate is then decanted from the dark oil and cooled in a freezer overnight to precipitate 0.7 g 205-12°C mp; 40% yield. ¹H NMR (d₆-DMSO. 300MHz) δ 8.08 (s, 1H), 8.06 (d, 1H), 7.60 (s, 1H), 7.45 (d, 1H), 5.48 (bs, 1 H), 4.65 (s, 2H), 3.35 (bs, 1 H). EI MS, [M]⁺=176.

### C. 4-Chloro-7-chloromethylquinazoline.

A mixture of 2.0 g (11.3 mmole) of 7-hydroxymethylquinazolin-4-one in 25 ml of phosphorus oxychloride is heated under reflux for 30 minutes. A very thick mixture is formed and the heating is continued for an additional 1.5 hours to give a solution. The phosphorus oxychloride is evaporated in a rotary evaporator and the residue is poured into ice water. The mixture is extracted with ether. The ether is dried over MgSO₄, filtered, and the filtrate evaporated. The residue is treated with 10 ml of ether and filtered. The filtrate is evaporated to afford 0.8 g of intermediate product which is used directly in the next step without further purification; 33% yield. ¹H NMR (CDCl₃, 300MHz) δ 9.07 (s, 1H). 8.30 (d, 1H), 8.06 (s, 1H), 7.78 (d, 1H), 4.78 (s, 2H). EI MS, [M]⁺=212, 214, 216 (Cl₂ pattern).

### D. 4-Amino-7-chloromethylquinazoline.

To 15 ml of a saturated ethanolic ammonia solution is added 1.0 g (4.7 mmole) of 4-chloro-7-chloromethylquinazoline. The mixture is stirred at room temperature overnight. The precipitate which forms is collected to give 0.7 g of the title intermediate product, mp>300°C; 77% yield. ¹H NMR (d₆-DMSO, 300MHz) δ 8.38 (s, 1H), 8.20 (d, 1H), 7.78 (bs, 2H), 7.70 (s, 1H), 7.51 (d, 1H), 4.92 (s, 2H). EI MS, [M]⁺=193, 195 (Cl pattern).

### E. 3-(4-Chloro-phenyl)-(E)-propenal.

To a solution of 3-(4-chloro-phenyl)-prop-2-(E)-en-1-ol (2.33 g, 13.8 mmol, prepared as described in J. Med. Chem. 1997, 1827) in 50 ml of CH₂Cl₂ is added activated manganese (IV) oxide (4.80 g , 55.3 mmol) in three portions over 3 hours and the resulting suspension is stirred at room temperature overnight. After filtration through a pad of celite and concentration *in vacuo,* the crude residue is purified by column chromatography eluting with 10% EtOAc/hexanes to provide the title intermediate compound (0.80 g , 4.80 mmol) as a pale yellow oil. ¹H NMR (CDCl₃, 300MHz) δ 9.71 (d, 1H), 7.48 (m, 3H), 7.41 (dd, 2H), 6.68 (dd, 1H).

### F. {2-[3-(4-Chloro-phenyl)-allylamino]-ethyl}-carbamic acid tert-butyl ester.

To a solution of N-Boc-ethylenediamine (0.63 g, 4.80 mmol) in 20mL of MeOH is added 3-(4-chloro-phenyl)-(E)-propenal (0.80 g, 4.80 mmol). After stirring for 3 hours at room temperature over 4A molecular sieves, NaBH₄ (0.19 g, 5.00 mmol) is added. The reaction mixture is stirred for 16 hours, then diluted with EtOAc and filtered through Celite plug. The solution is concentrated under reduced pressure. The residue is partitioned between EtOAc and H₂O and the layers are separated. The aqueous layer is extracted with EtOAc. The combined organic layers are washed with H₂O, brine, then dried over MgSO₄, filtered and concentrated. The crude title product is purified by column chromatography, eluting with a gradient of 25% EtOAc/CH₂Cl₂ to 50% EtOAc/CH₂Cl₂, to provide the title intermediate compound (0.80g, 2.57 mmol). ¹H NMR (CDCl₃, 300MHz) δ 7.26 (s, 4H), 6.49 (d, 1 H), 6.23 (dt, 1H), 4.96 (bs, 1H), 3.40 (m, 2H), 3.25 (m, 2H), 2.76 (m, 2H), 1.60 (bs, 1H), 1.45 (s, 9H).

### G. N-(2-tert-Butoxycarbonylamino-ethyl)-N-13-(4-chloro-phenyl)-allyl]-oxalamic acid methyl ester.

To a solution of {2-[3-(4-chloro-phenyl)-allylamino]-ethyl}-carbamic acid tert-butyl ester (0.80 g, 2.57 mmol) in 15 ml of CH₂Cl₂ at 0°C is added triethylamine (0.54 mL , 3.85 mmol) and methyl chlorooxoacatate (0.25 mL, 2.70 mmol). The resulting mixture is stirred at 0°C for 1 h, then at room temperature for 1 h. The solution is partitioned between EtOAc and H₂O and the layers separated. The organic layer is washed with 1 N HCl solution, H₂O, saturated NaHCO₃ solution and brine, then dried over MgSO_{4,} filtered and concentrated. The crude product is purified by column chromatography eluting with 25% EtOAc/CH₂Cl₂ to provide the title intermediate compound (0.98g, 2.47 mmol). ¹H NMR (CDCl₃, 300MHz) δ 7.31 (m, 4H), 6.55 (dd, 1H), 6.14 (m, 1 H), 4.88 (bs, 1H), 4.21, 4.10 (d, 2H, rotamers), 3.91, 3.86 (s, 3H, rotamers), 3.55, 3.44 (m, 2H, rotamers), 3.36 (m, 2H), 1.43 (s, 9H).

### H. 1-[3-(4-Chloro-phenyl)-allyl]-piperazine-2,3-dione.

A solution of N-(2-tert-butoxycarbonylamino-ethyl)-N-[3-(4-chloro-phenyl)-allyl]-oxalamic acid methyl ester (0.49 g, 1.23 mmol) in 6 mL of EtOAc at 0 °C is saturated with HCl gas. The ice-bath is removed and the solution is stirred at room temperature for 30 min as a white precipitate forms after about 5-10 min. After this time, the solution is concentrated to a white solid (0.41 g). The crude amine salt is suspended in 6 mL CH₂Cl₂ and 1.5 mL of MeOH. Triethylamine (0.5 mL, 3.53 mmol) is added and the resulting solution is stirred at room temperature overnight. The solution is concentrated under reduced pressure and partitioned between CH₂Cl₂ and H₂O. The aqueous layer is basified with 0.5N NaOH. The organic layer is washed with H₂O, brine, then dried over MgSO₄, filtered and concentrated. The title intermediate compound is obtained as a white solid (0.32 g, 1.21 mmol). ¹H NMR (CDCl₃, 300MHz) δ 7.82 (bs, 1H), 7.30 (s, 4H), 6.56 (d, 1H), 6.14 (dt, 1H), 4.27 (d, 2H), 3.58 (m, 4H).

### I. 1-(4-Amino-quinazolin-7-ylmethyl)-4-[3-(4-chloro-phenyl)-allyl]-piperazine-2,3-dione.

To a solution of 1-[3-(4-chloro-phenyl)-allyl]-piperazine-2,3-dione (60 mg, 0.23 mmol) in 1.5 mL of DMF is added NaH (10 mg of a 60% dispersion in mineral oil, 0.24 mmol). The mixture is heated at 55°C for 20 min. To the solution is added 4-amino-7-chloromethyl-quinazoline (49 mg, 0.25 mmol), and the resulting mixture is heated at 55°C for 20 min as a white precipitate is formed. After this time, reaction mixture is quenched with a few drops of H₂O and MeOH, then concentrated. The crude product is purified by RP-HPLC, eluting in a gradient of 10% CH₃CN/H₂O (0.1% TFA) to 70% CH₃CN/H₂O (0.1% TFA) over 30 minutes, and the appropriate product fractions are combined and lyopholized to provide the title compound (56 mg, 0.10 mmol) as a white solid. ¹H NMR (d₆₋DMSO, 300 MHz) δ 9.78 (bs, 2H), 8.83 (s, 1 H), 8.40 (s, 1H), 7.72 (d, 1H), 7.65 (s, 1H), 7.49 (d, 2H), 7.38 (d, 2H), 6.61 (d, 1 H), 6.30 (dt, 1 H), 4.80 (s, 2H), 4.18 (d, 2H), 3.59 (m, 4H). ISP MS, [M+H]⁺=422.

### Reference EXAMPLE 1328. 1-[3-(5-chloro-thiophen-2-yl)-allyl]-4-(4-pyridin-4-yl-benzyl)-piperazine-2,3-dione.

### A. {2-[3-(5-Chloro-thiophen-2-yl)-allylamino]-ethyl}-carbamic acid tert-butyl ester.

The title compound is prepared as described in EXAMPLE 1306, Part F from 3-(5-chloro-thiophen-2-yl)-(E)-propenal. ¹H NMR (CDCl₃, 300MHz) δ 6.77 (d, 1H), 6.67 (d, 1H), 6.52 (d, 1H), 5.99 (dt, 1H), 4.95 (bs, 1H), 3.37 (m, 2H), 3.24 (m, 2H), 2.76 (m, 2H), 1.48 (bs, 1H), 1.45 (s, 9H).

### B. N-1-[3-(5-Chloro-thiophen-2-yl)-allylamino]-ethane-1,2-diamine hydrochloride.

A solution of {2-[3-(5-chloro-thiophen-2-yl)-allylamino]-ethyl}-carbamic acid tert-butyl ester (0.11 g, 0.35 mmol) in 20 mL of EtOAc at 0 °C is saturated with HCl gas. The ice-bath is removed and the solution is stirred at room temperature for 1 h. After this time, the solution is concentrated to a white solid (0.41 g). The title compound is obtained as a white solid (0.07 g, 0.27 mmol) and used as is in the following step. ¹H NMR (CDCl₃, 300MHz) δ 6.76 (d, 1 H), 6.68 (d, 1H), 6.54 (d, 1H), 6.01 (dt, 1H), 3.38 (m, 2H), 2.82 (m, 2H), 2.71 (m, 2H), 1.40 (bs, 3H).

### C. N-[3-(5-Chloro-thiophen-2-yl)-allyl]-N'-(4-pyridin-4-yl-benzyl)-ethane-1,2-diamine.

To a solution of N-1-[3-(5-chloro-thiophen-2-yl)-allylamino]-ethane-1,2-diamine hydrochloride (0.07 g, 0.27 mmol) in 10mL of MeOH is added 4-pyridin-4-yl-benzaldehyde (0.05 g, 0.27 mmol). After stirring for 16 h at room temperature over 4A molecular sieves, NaBH₄ (0.01 g, 0.27 mmol) is added. The reaction mixture is stirred for 5 h, filtered through a Celite plug and concentrated. The residue is partitioned between EtOAc and H₂O and the layers are separated. The aqueous layer is extracted with EtOAc. The combined organic layers are washed with H₂O (3X), brine, then dried over MgSO₄, filtered and concentrated. The crude material is purified by column chromatography, eluting with a gradient of 5% MeOH/CH₂Cl₂ to 10% MeOH/CH₂Cl₂ with 2% NH₄OH present to provide the title compound (0.042g, 0.11 mmol). ¹H NMR (CDCl₃, 300MHz) δ 8.65 (d, 2H), 7.61, (d, 2H), 7.50 (d, 2H), 7.45 (d, 2H), 6.76 (d, 1H), 6.67 (d, 1H), 6.52 (d, 1H), 6.00 (dt, 1H), 3.38 (s, 2H), 3.35 (d, 2H), 2.78 (s, 4H), 1.78 (bs, 2H).

### D. 1-13-(5-chloro-thiophen-2-yl)-allyl]-4-(4-pyridin-4-yl-benzyl)-piperazine-2,3-dione.

To a solution of N-[3-(5-Chloro-thiophen-2-yl)-allyl]-N'-(4-pyridin-4-yl-benzyl)-ethane-1,2-diamine (0.037 g, 0.10 mmol) in 1.5 ml of EtOH is added dimethyl oxalate (0.012 g, 0.10 mmol). The resulting mixture is stirred at room temperature for 16 h, then heated at 50°C for 16 h. The solution is concentrated. The crude product is purified by column chromatography, eluting with a gradient of 5% MeOH/CH₂Cl₂ to 10% MeOH/CH₂Cl₂ to provide the title compound as a white solid (0.024 g, 0.04 mmol). ¹H NMR (CDCl₃, 300MHz) δ 8.68 (d, 2H), 7.63, (d, 2H), 7.50 (m, 2H), 7.43 (d, 2H), 6.79 (d, 1H), 6.672(d, 1H), 6.59 (d, 1 H), 5.87 (dt, 1H), 4.76 (s, 2H), 4.20 (d, 2H), 3.48 (s, 4H). ISP MS, [M+H]⁺=438, 440, Cl pattern.

The following 2,3-diketopiperazine compounds are prepared in a similar fashion using the procedures described above.

| Example | Name | m/z [M+H] |
|---|---|---|
| 1361 | 1-(4-Amino-quinazolin-7-ylmethyl)-4-[5-(5-chloro-thiophen-2-yl)-isoxazol-3-ylmethyl]-piperazine-2,3-dione | ISP-469, Cl |

### Example 1523 (R)-1-(4-Amino-quinazolin-7-ylmethyl)-4-[5-(5-chloro-thiophen-2-yl)-isoxazol-3-ylmethyl]-6-methoxymethyl-piperazin-2-one

A: (*R*)-4-Benzyl-6-(tert-butyl-dimethyl-silanyloxymethyl)-piperazin-2-one. A solution of (*R*)-4-benzyl-6-hydroxymethyl-piperazin-2-one (9 g, 41 mmol) and imidazole (4.2 g, 62 mmol) in CH₂Cl₂ (80 mL) at 0 °C was treated with TBSCI (6.24 g, 42 mmol). After 1 h at ambient temperature, the mixture was concentrated, diluted with EtOAc, and filtered to remove most of the triethylamine hydrochloride. Concentration of the solution provided 12.8 g (93%) of the crude (*R*)-4-benzyl-6-(tert-butyl-dimethyl-silanyloxymethyl)-piperazin-2-one which was used for the next reaction without further purification. Mass Spectrum: (ESI) *m*/*z* 335 (M+H)⁺.
B: (*R*)-6-(tert-Butyl-dimethyl-silanyloxymethyl)-piperazin-2-one. To a solution of (R)-4-benzyl-6-(tert-butyl-dimethyl-silanyloxymethyl)-piperazin-2-one (2.1 g, 6.3 mmol) in MeOH (80 mL) was added 10% palladium hydroxide on C (0.3 g). The reaction mixture was hydrogenated at ambient temperature under a balloon of H₂ for 20 h. The reaction mixture was filtered through a pad of celite, and the filtrate was concentrated to afford 1.36 g (88%) of (*R*)-6-(tert-butyl-dimethyl-silanyloxymethyl)-piperazin-2-one. Mass Spectrum (ESI) *m*/*z* 245 (M+H)⁺.
C: (*R*)-6-(tert-Butyl-dimethyl-silanyloxymethyl)-4-[5-(5-chloro-thiophen-2-yl)-isoxazol-3-ylmethyl]-piperazin-2-one. To a mixture of (R)-6-(tert-butyl-dimethyl-silanyloxymethyl)-piperazin-2-one (1.36 g, 5.6 mmol) and K₂CO₃ (3.1 g, 22 mmol) in anhydrous DMF (10 mL) at 0 °C was added 3-bromomethyl-5-(5-chloro-thiophen-2-yl)-isoxazole (1.72 g, 6.2 mmol). After 2 h at ambient temperature, the reaction mixture was diluted with EtOAc and water, and the layers were separated. The organic phase was washed twice with water, brine, dried (MgSO₄), filtered and concentrated. The crude residue was chromatographed on SiO₂ (CH₂Cl₂ to 10% MeOH in CH₂Cl₂) to provide 2.55 g (100%) of (*R*)-6-(tert-butyl-dimethyl-silanyloxymethyl)-4-[5-(5-chloro-thiophen-2-yl)-isoxazol-3-ylmethyl]-piperazin-2-one.
   ¹H NMR (300 MHz, CDCl₃) δ⁻ 7.27 (d, *J* = 4.2 Hz, 1H), 6.95 (d, *J* = 4.2 Hz, 1H), 6.35 (s, 1H), 6.09 (s, 1 H), 3.7-3.45 (m, 5H), 3.33 (d, *J* = 16 Hz, 1 H), 3.14 (d, *J* = 16 Hz, 1H), 2.76 (dd, *J* = 15, 3.6 Hz, 1H), 2.4-2.33 (m, 1 H), 0.87 (s, 9H), 0.05 (s, 6H) ppm. Mass Spectrum: (ESI) *m*/*z* 442 (M+H)⁺,
D: (*R*)-2-(Benzhydrylidene-amino)-4-{2-(tert-butyl-dimethyl-silanyloxymethyl)-4-[5-(5-chloro-thiophen-2-yl)-isoxazol-3-ylmethyl]-6-oxo-piperazin-1-ylmethyl}-benzonitrile. To a solution of (*R*)-6-(tert-butyl-dimethyl-silanyloxymethyl)-4-[5-(5-chloro-thiophen-2-yl)-isoxazol-3-ylmethyl]-piperazin-2-one (2.55 g, 5.7 mmol) in a mixture of 5:1 THF:DMF (25 mL) at 0 °C was added NaH (a 60% dispersion in mineral oil, 0.32 g, 8 mmol) followed by 2-(benzhydrylidene-amino)-4-bromomethyl-benzonitrile (2.35 g, 6.3 mmol). After 0.5 h at 0 °C, the reaction mixture was diluted with saturated NH₄Cl, and extracted with EtOAc. The combined organic extracts were washed with water (twice), dried (MgSO₄), filtered and concentrated. The crude (*R*)-2-(benzhydrylidene-amino)-4-{2-(tert-butyl-dimethyl-silanyloxymethyl)-4-[5-(5-chloro-thiophen-2-yl)-isoxazol-3-ylmethyl]-6-oxo-piperazin-1-ylmethyl}-benzonitrile was used directly in the next reaction without further purification.
E: (*R*)-2-(Benzhydrylidene-amino)-4-{4-[5-(5-chloro-thiophen-2-yl)-isoxazol-3-ylmethyl]-2-hydroxymethyl-6-oxo-piperazin-1-ylmethyl}-benzonitrile. A solution of (R)-2-(benzhydrylidene-amino)-4-{2-(tert-butyl-dimethyl-silanyloxymethyl)-4-[5-(5-chloro-thiophen-2-yl)-isoxazol-3-ylmethyl]-6-oxo-piperazin-1-ylmethyl}-benzonitrile in THF (30 mL) was treated with a 1.0 M solution of TBAF in THF (7 mL, 7 mmol). The reaction mixture was stirred for 0.5 h at ambient temperature, concentrated and chromatographed on SiO₂ (CH₂Cl₂ to 4% MeOH in CH₂Cl₂) to yield 3.3 g (95%) of (*R*)-2-(benzhydrylidene-amino)-4-{4-[5-(5-chloro-thiophen-2-yl)-isoxazol-3-ylmethyl]-2-hydroxymethyl-6-oxo-piperazin-1-ylmethyl}-benzonitrile.
   ¹H NMR (300 MHz, CDCl₃) δ⁻ 7.79 (d, *J* = 7.2 Hz, 2H), 7.6-7.2 (m, 10 H), 6.95 (d, *J* = 4.1 Hz, 1H), 6.90 (d, *J* = 6.9 Hz, 1H), 6.62 (s, 1H), 6.31 (s, 1H), 5.15 (d, *J* = 15.4 Hz, 1H), 3.96 (d, *J* = 15.4 Hz, 1H), 3.75-3.6 (m, 5H), 3.2-2.9 (m, 3H), 2.51 (dd, J = 11.7, 2.8 Hz, 1 H) ppm. Mass Spectrum: (ESI) *m*/*z* 622 (M+H)⁺.
F: (*R*)-2-(Benzhydrylidene-amino)-4-{4-[5-(5-chloro-thiophen-2-yl)-isoxazol-3-ylmethyl]-2-methoxymethyl-6-oxo-piperazin-1-ylmethyl}-benzonitrile. To a solution of (*R*)-2-(benzhydrylidene-amino)-4-{4-[5-(5-chloro-thiophen-2-yl)-isoxazol-3-ylmethyl]-2-hydroxymethyl-6-oxo-piperazin-1-ylmethyl}-benzonitrile (0.15 g, 0.24 mmol) in THF/DMF (5:1, 6 mL) at 0 °C were added 60% NaH in mineral oil (26 mg, 0.68 mmol), followed, after 15 min, by the addition of Mel (0.018 mL, 0.29 mmol). After 1 h at ambient temperature, the mixture was quenched with saturated aqueous NH₄Cl solution and extracted with EtOAc. The combined organic extracts were dried (MgSO₄), filtered, and concentrated. The crude (R)-2-(benzhydrylidene-amino)-4-{4-[5-(5-chloro-thiophen-2-yl)-isoxazol-3-ylmethyl]-2-methoxymethyl-6-oxo-piperazin-1-ylmethyl}-benzonitrile was used directly in the next reaction without further purification.
G: (*R*)-2-Amino-4-{4-[5-(5-chloro-thiophen-2-yl)-isoxazol-3-ylmethyl]-2-methoxymethyl-6-oxo-piperazin-1-ylmethyl}-benzonitrile. Concentrated HCl (12M, 5 drops) was added at 0 °C to a solution of (*R*)-2-(benzhydrylidene-amino)-4-{4-[5-(5-chloro-thiophen-2-yl)-isoxazol-3-ylmethyl]-2-methoxymethyl-6-oxo-piperazin-1-ylmethyl}-benzonitrile in MeOH (5 mL). After 1 h at ambient temperature, the reaction mixture was concentrated and partitioned between EtOAc and saturated NaHCO₃ solution. The separated organic phase was washed with brine, dried (MgSO₄), filtered and concentrated. The crude residue was chromatographed on silica gel (CH₂Cl₂ to 5% MeOH in CH₂Cl₂) to provide 90 mg (79%) of (*R*)-2-amino-4-{4-[5-(5-chloro-thiophen-2-yl)-isoxazol-3-ylmethyl]-2-methoxymethyl-6-oxo-piperazin-1-ylmethyl}-benzonitrile. Mass Spectrum: (ESI) *m*/*z* 472 (M+H)⁺.
H: (*R*)-1-(4-Amino-quinazolin-7-ylmethyl)-4-[5-(5-chloro-thiophen-2-yl)-isoxazol-3-ylmethyl]-6-methoxymethyl-piperazin-2-one (RPR257852A). To a solution of (*R*)-2-amino-4-{4-[5-(5-chloro-thiophen-2-yl)-isoxazol-3-ylmethyl]-2-methoxymethyl-6-oxo-piperazin-1-ylmethyl}-benzonitrile (90 mg, 0.19 mmol) in absolute ethanol (3 mL) was added 1,3,5-triazine (0.16 g, 1.9 mmol) and acetic acid (0.11 mL, 1.9 mmol). The solution was heated to reflux. After 15 h, the solution was concentrated and diluted with water and acetonitrile to make 10 mL of solution. The crude material was purified by RP-HPLC eluting with a gradient of 10% CH₃CN/H₂O (0.1% TFA) to 80% CH₃CN/H₂O (0.1% TFA) and the appropriate product fractions were combined and lyophilized to give 75 mg (79%) of (*R*)-1-(4-amino-quinazolin-7-ylmethyl)-4-[5-(5-chloro-thiophen-2-yl)-isoxazol-3-ylmethyl]-6-methoxymethyl-piperazin-2-one as a white solid. Mass Spectrum: (ESI) *m*/*z* 499 (M+H)⁺.

The following compounds were prepared by the O-alkylation reaction of the C(6)-hydroxymethyl templates with appropriate electrophiles as described in above examples.

| Example | Compound Name | *m*/*z* (M+H) |
|---|---|---|
| 1524 | (6R)-1-(4-Amino-quinazolin-7-ylmethyl)-4-[5-(5-chloro-thiophen-2-yl)-isoxazol-3-ylmethyl]-6-ethoxymethyl-piperazin-2-one | 513 |

### Preparations of Methylhalides

### Preparation of 3-Bromomethyl-5-(5-chloro-thiophen-2-yl)-isoxazole

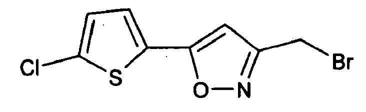

### Part A. 4-(5-Chloro-thiophen-2-yl)-2,4-dioxo-butyric acid ethyl ester.

Sodium hydride (60% dispersion in mineral oil, 2.43 g, 60.8 mmol) was added to the solution of 2-acetyl-5-chlorothiophene (4.88 g, 30.4 mmol) in 150 ml of anhydrous toluene at 0°C under nitrogen in two portions. After the mixture was stirred at 0 °C for 10 minutes, diethyl oxalate (6.20 ml, 45.6 mmol) was added via syringe, the resulting mixture was stirred at 0 °C for half an hour, then heated to 140 °C gradually and refluxed for 1 hour. The solvents were removed under reduced pressure, 250 ml of water was added to the residue which was then washed with ethyl acetate ( 75 ml x 2), the aqueous portion was cooled to 0°C and to which was added 2N HCl till PH = 2, ethyl acetate was used to extract the acidified aqueous portion (100 ml x 4), the combined organic portions were then washed with brine, dried with MgSO₄. Removal of the solvents afforded the desired product as a brownish solid, 7.10 g, which was used in the following reaction without further purification, 90%. ¹H NMR (CD₃OD) δ1.36 (t, 3H, J = 7.1 Hz), 4.35 (q, 2H, J = 7.1 Hz), 6.95 (s, 1H), 7.15 (m, 1H), 7.87 (m, 1H).

### Part B. 5-(5-chloro-thiophen-2-yl)-isoxazole-3-carboxylic acid ethyl ester.

The mixture of -(5-Chloro-thiophen-2-yl)-2,4-dioxo-butyric acid ethyl ester (5.40 g, 20.7 mmol) and hydroxylamine hydrochloride (5.04 g, 72.5 mmol) in 150 ml of anhydrous ethanol was refluxed for three hours. The solvents were removed under reduced pressure, 150 ml of H2O was added to the residue, followed by ammonium hydroxide (28-30 %) till PH=7. The aqueous mixture was then extracted with ethyl acetate (75ml x3 ), the combined organic portions were washed with brine, dried with MgSO₄. After the solvents were removed, the crude product was purified flash column chromatography (10% of ethyl acetate/hexanes), 4.35 g of the desired product was obtained as a pale solid, 82%. ¹H NMR (CDCl₃) δ 1.41 (t, 3H, J = 7.1 Hz), 4.44 (q, 2H, J = 7.1 Hz), 6.71 (s, 1H), 6.95 (d, 1H. J = 4.0 Hz), 7.31 (d, 1H, J = 4.0 Hz).

### Part C. [5-(5-chloro-thiophen-2-yl)-isoxazol-3-yl]-methanol.

Sodium borohydride (3.20 g, 84.4 mmol) was added to the solution of 5-(5-chloro-thiophen-2-yl)-isoxazole-3-carboxylic acid ethyl ester (4.35 g, 16.9 mmol) in 80 ml of anhydrous ethanol at 0 °C, the mixture was then stirred at room temperature for 12 hours. Ethanol was removed under reduced pressure, the residue was taken up in H₂O (100ml), and NH₄Cl (aq) was added to consume the excess NaBH₄. The product was extracted with ethyl acetate (75ml x 3), the combined organic portions were washed with brine, dried with MgSO₄, removal of the solvents afforded 3.53 g of white solid as the desired product, 97%. ¹H NMR (CDCl₃) δ 4.78 (s, 2H), 6.40 (s, 1 H), 6.94 (d, 1H, J = 3.9 Hz), 7.27 (d, 1H, J = 3.9 Hz).

### Part D. 3-Bromomethyl-5-(5-chloro-thiophen-2-yl)-isoxazole.

NBS (3.19 g, 17.9 mmol) was added to the mixture of 5-(5-chloro-thiophen-2-yl)-isoxazol-3-yl]-methanol (3.50 g, 16.2 mmol) and triphenyl phosphine (4.68 g, 17.9 mmol) in 80 ml of anhydrous methylene chloride at 0 °C. The mixture was then stirred at room temperature for 1 hour. The solvents were removed, the residue was purified by flash column chromatography (10 % of ethyl acetate/hexanes), 4.0 g of the product was obtained as a white solid, 89%. ¹H NMR (CDCl₃) δ 4.42 (s, 2H), 6.42 (s, 1H), 6.95 (d, 1H, J = 4.0 Hz), 7.28 (d, 1 H, J = 4.0 Hz).

### Preparation of 3-Bromomethyl-5-(3-chloro-phenyl)-isoxazole

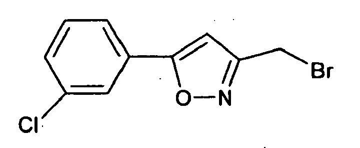

3-Bromomethyl-5-(3-chloro-phenyl)-isoxazole was prepared according to the methods described for 3-methyl-5=(5-chloro-thiophen-2-yl)-1 H-pyrazole. ¹H NMR (CDCl₃) δ 4.46 (s, 2H), 6.64 (s, 1 H), 7.41-7.43 (m, 2H), 7.64-7.67 (m, 1 H), 7.77 (m, 1 H).

### Preparation of 3-Bromomethyl-5-(4-chlorophenyl)-isoxazole

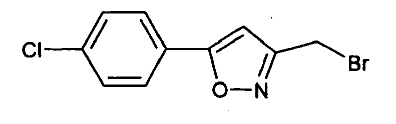

3-Bromomethyl-5-(4-chlorophenyl)-isoxazole was prepared according to the methods described for 3-methyl-5-(5-chloro-thiophen-2-yl)-1H-pyrazole. ¹H NMR (CDCl₃) δ 4.45 (s, 2H), 6.60 (s, 1H), 7.44 (d, 2H, J=8.6 Hz), 7.70(d, 2H, J= 8.6 Hz).

### Preparation of 5-bromomethyl-3-(5-chloro-thiophen-2-yl)-isoxazole

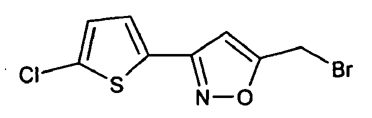

### Method I:

### Part A. 5.-Chloro-thiophene-2-carbaldehyde oxime.

A mixture of 5-chloro-2-thiophene carboxaldehyde (1.36 g, 9.28 mmol) and hydroxyamine hydrochloride (0.71 g, 10.2 mmol) in 8 ml of pyridine was stirred at room temperature overnight. Pyridine was removed under reduced pressure, the residue was purified by flash column chromatography (5% -10% of ethyl acetate in methylene chloride), 1.02 g of the product was obtained as a white solid, 68%. ¹H NMR (CD₃OD) δ 6.94 (d, 1 H, J=4.0 Hz), 7.14 (d, 1H, J=4.0 Hz), 7.60 (s, 1 H), 8.81 (broad, 1 H, OH).

### Part B. 5-Bromomethyl-3-(5-chloro-thioahen-2-yl)-isoxazole

N-chlorosuccinimide (0.28 g, 2.10 mmol) was added to the solution of 5-Chloro-thiophene-2-carbaldehyde oxime (0.33 g, 2.04 mmol) in 10 ml of anhydrous DMF at room temperature under N₂, followed by the addition of two drops of pyridine. The mixture was stirred at r.t. for one hour, at 60 °C for 3 hours, the mixture was cooled to 0°C and to which was added propargyl bromide (80 wt. % in toluene, 2.30 ml, 20.4 mmol), a solution of triethyl amine (0.29 ml, 2.04 mmol) in 2.5 ml of DMF was then added dropwise slowly in a period of 25 minutes . The reaction was warmed to room temperature and stirred for 18 hours, diluted with water (200 ml), extracted with ethyl acetate (50ml x 3), the combined organic portions were washed with water (100 ml x 2), brine, dried with MgSO₄. After the solvents were removed, the crude product was purified flash column chromatography (5%-10% of ethyl acetate/hexanes), 0.28 g of the desired product was obtained as a white solid, 49%. ¹H NMR (CDCl₃) δ 4.46 (s, 2H), 6.51 (s, 1H), 6.92 (d, 1H, J = 3.9 Hz), 7.19 (d, 1H, J = 3.9 Hz).

### Method II:

### Part A. 3-(5-chloro-thiopfien-2-yl)-isoxazole-5-carboxylic acid ethyl ester.

N-chlorosuccinimide (0.32 g, 2.41 mmol) was added to the solution of 5-Chloro-thiophene-2-carbaldehyde oxime (0.39 g, 2.41 mmol) in 10 ml of anhydrous DMF at room temperature under N₂, followed by the addition of two drops of pyridine. After stirred at 60 °C for 3 hours, the mixture was cooled to 0 °C and to which was added methyl propiolate (1.0 ml, 12.1 mmol), a solution of triethyl amine (0.34 ml, 2.41 mmol) in 2.5 ml of DMF was then added slowly in a period of 30 minutes . The reaction was warmed to room temperature and stirred for 12 hours, diluted with water (200 ml), extracted with ethyl acetate (50ml x 2), the combined organic portions were washed with water, brine, dried with MgSO₄. After the solvents were removed, the crude product was purified flash column chromatography (3%-10% of ethyl acetate/hexanes), 0.19 g of the desired product was obtained as a white solid, 32%. ¹H NMR (CDCl₃) δ 3.99 (s, 3H), 6.95 (d, 1H, J=3.9 Hz), 7.11 (s, 1H), 7.26 (d, 1 H, J=3.9 Hz).

### Part B. [3-(5-chloro-thiophen-2-yl)-isoxazol-5-yl]-methanol.

Sodium borohydride (0.14 g, 3.90 mmol) was added to a suspension of 3-(5-chloro-thiophen-2-yl)-isoxazole-5-carboxylic acid ethyl ester (0.19 g, 0.78 mmol) in 15 ml of anhydrous methanol at 0 °C. The mixture was then stirred at 0 °C under N₂, at room temperature for half an hour. Methanol was removed under reduced pressure, the residue was taken up in H₂O (50ml), and NH₄Cl (aq) was added until pH = 7 was obtained. The product was extracted with ethyl acetate (30 ml x 2), the combined organic portions were washed with brine, dried with MgSO₄, removal of the solvents afforded 0.14 g of white solid as the desired product, 83%. ¹H NMR (CDCl₃) δ 4.79 (s, 2H), 6.44 (s, 1 H), 6.92 (d, 1 H, J = 3.9 Hz), 7.19 (d, 1 H, J = 3.9 Hz).

### Part C. 3-Bromomethyl-5-(5-chloro-thiophen-2-yl)-isoxazole.

N-bromosuccinimide (0.14 g, 0.78 mmol) was added to the mixture of [3-(5-chloro-thiophen-2-yl)-isoxazol-5-yl]-methanol (0.14 g, 0.65 mmol) and triphenyl phosphine (0.21 g, 0.78 mmol) in 15 ml of anhydrous methylene chloride at 0 °C under N₂. The mixture was then stirred at room temperature for 1 hour. The solvents was removed, the residue was purified by flash column chromatography (10 % of ethyl acetate/hexanes), 0.13 g of the product was obtained as a white solid, 72%. ¹H NMR (CDCl₃) δ 4.46 (s, 2H), 6.51 (s, 1 H), 6.92 (d, 1 H, J = 3.9 Hz), 7.19 (d, 1 H, J = 3.9 Hz).

Using the methylhalides described in the preparations and the methods of the previous examples the following inhibitors were prepared:

### Example 1535 1-(4-Amino-quinazolin-7-ylmethyl)-4-[5-(5-chloro-thiophen-2-yl)-isoxazol-3-ylmethyl]-3-(S)-propyl-piperazin-2-one ditrifluoroacetate.

¹H NMR (300 MHz, DMSO) δ 9.74 (2H, bs), 8.81 (1H, s), 8.37 (1 H, d, J = 8.5 Hz), 7.54 - 7.61 (3H, m), 7.28 - 7.29 (1H, m), 6.88 (1H, s), 4.64 - 4.76 (2H, m), 3.91 (1 H, d, J = 14.4 Hz), 3.71 (1H, d, J = 14.4 Hz), 3.27 - 3.30 (2H, m), 3.13 91H, t, J = 4.7 Hz), 3.01 - 3.05 (1H, m), 2.61 - 2.65 (1 H, m), 1.80 - 1.86 (2H, m), 1.39 -1.52 (1 H, m), 1.13 - 1.27 (1 H, m), 0.80 - 0.85 (3H, m). MS (Ion spray) [M+H]⁺ of 497/499 observed, chloro pattern.

### Example 1536 1-(4-Amino-quinazolin-7-ylmethyl)-4-[5-(5-chloro-thiophen-2-yl)-isoxazol-3-ylmethyl]-3-(S)-methyl-piperazin-2-one ditrifluoroacetate.

¹H NMR (300 MHz, DMSO) δ 9.76 (2H, bs), 8.81 (1 H, s), 8.36 (1 H, d, J = 8.6 Hz), 7.59 - 7.61 (2H, m), 7.52 (1 H, s), 7.29 (1 H, d, J = 4.0 Hz), 6.92 (1 H, s), 4.63 - 4.70 (2H, m), 3.95 (1H, d, J = 14.5 Hz), 3.76 (1 H, d, J = 14.5 Hz), 3.24 - 3.32 (3H, m), 2.97 - 3.05 (1H, m), 2.60 - 2.70 (1 H, m), 1.40 (3H, d, J = 6.8 Hz). MS (Ion spray) [M+H]⁺ of 469/471 observed, chloro pattern.

### Example 1537 1-(4-Amino-quinazolin-7-ylmethyl)-4-[3-(5-chloro-thiophen-2-yl)-isoxazol-5-ylmethyl]- piperazin-2-one ditrifluoroacetate.

¹H NMR (300 MHz, DMSO) δ 9.81 (1H, bs), 9.77 (1H, s), 8.80 (1H, s), 8.38 (1H, d, J = 8.5 Hz), 7.59 - 7.62 (2H, m), 7.24 (1 H, d, J = 3.9 Hz), 7.04 (1 H, s), 4.71 (2H, s), 3.91 (2H, s), 3.30 - 3.34 (4H, m), 2.82 - 2.85 (2H, m). MS (Ion spray) [M+H]⁺ of 455/457 observed, chloro pattern.

### Example 1539 1-(4-Amino-quinazolin-7-ylmethyl)-4-[3-(5-chloro-thiophen-2-yl)-isoxazol-5-ylmethyl]-3- methyl-piperazin-2-one ditrifluoroacetate.

¹H NMR (300 MHz, DMSO) δ 9.76 (2H, bs), 8.81 (1H, s), 8.36 (1 H, d, J = 8.6 Hz), 7.59 - 7.62 (2H, m), 7.51 (1H, s), 7.25 (1H, d, J = 3.9 Hz), 7.03 (1H, s), 4.69 (2H, s), 4.05 (1H, d, J = 15.6 Hz), 3.94 (1 H, d, J = 15.5 Hz), 3.34 - 3.36 (1 H, m), 3.20 - 3.26 (2H, m), 3.04 - 3.08 (1H, m), 2.65 - 2.71 (1 H, m), 1.40 (3H, d, J = 6.8 Hz). MS (Ion spray) [M+H]⁺ of 469/471 observed, chloro pattern.

### Example 1558 1-4-Amino-quinazolin-7-ylmethyl)-4-[5-(5-chloro-thiophen-2-yl)-isoxazol-3-ylmethyl]-piperazin-2-one ditrifluoroacetate.

¹H NMR (300 MHz, DMSO) δ 9.75 (2H, bs), 8.81 (1H, s), 8.36 (1H, d, J = 8.6 Hz), 7.55 - 7.63 (3H, m), 7.29 (1H, d, J = 4.0 Hz), 6.92 (1H, s), 4.71 (2H, s), 3.74 (2H, s), 3.18 - 3.29 (4H, m), 2.76 - 2.78 (2H, m). MS (Ion spray) [M+H]⁺ of 455/457 observed, chloro pattern.

### Example 1560 4-[5-(5-Chloro-thiophen-2-yl)-isoxazol-3-ylmethyl]-1-(1H-pyrrolo[3,2-c]pyridin-2-ylmethyl)-piperazin-2-one ditrifluoroacetate

To a solution of 2-(2-oxo-piperazin-1-ylmethyl)-pyrrolo[3,2-c]pyridine-1-carboxylic acid tert-butyl ester (0.33g, 1.0 mmol) and Et₃N (0.34 mL, 2.4 mmol) in MeCN (40 mL) was added a solution of 3-bromomethyl-5-(5-chloro-thiophen-2-yl)-isoxazole in MeCN (10 mL). The solution was stirred at r.t. overnight and concentrated to dryness. The residue was then treated with 20% TFA/DCM (40 mL) overnight. The solution was concentrated. The residue was purified by RP HPLC to give the product as a white solid (0.23 g, 0.35 mmol). ¹H NMR (DMSO) δ 14.6 (br, 1H), 12.7 (s, 1H), 9.14 (s, 1 H), 8.36 (d, 1H), 7.86 (d, 1H), 7.61 (d, 1 H), 7.33 (d, 1H), 6.93 (s, 1H), 6.87 (s, 1H), 4.75 (s, 2H), 3.75 (s, 2H), 3.36 (t, 2H), 3.23 (s, 2H), 2.75 (t, 2H). MS M+1: 428, 430.

### Inhibition of Factor Xa

The compounds described herein inhibit blood coagulation by virtue of their ability to inhibit the penultimate enzyme in the coagulation cascade, controlling the activity of Factor Xa. Both the activity of free Factor Xa and Factor Xa assembled in the prothrombinase complex (Factor Xa, Factor Va, calcium and phospholipid) are inhibited by compounds described herein. The inhibition of the Factor Xa activity is obtained by direct complex formation between the inhibitor and the enzyme and is therefore independent of the plasma co-factor antithrombin III. Effective inhibition of the Factor Xa activity is achieved by administering the compounds either by oral administration, continuous intravenous infusion, bolus intravenous administration or any other parenteral route such that it achieves the desired effect of preventing the activity of Factor Xa induced formation of thrombin from prothrombin.

Anticoagulant therapy is indicated for the treatment and prophylaxis of a variety of thrombotic conditions of both the venous and arterial vasculature. In the arterial system, abnormal thrombus formation is primarily associated with arteries of the coronary, cerebral and peripheral vasculature. The diseases associated with thrombotic occlusion of these vessels principally include acute myocardial infarction (AMI), unstable angina, thromboembolism, acute vessel closure associated with thrombolytic therapy and percutaneous transluminal coronary angioplasty (PTCA), transient ischemic attacks, stroke, intermittent claudication and bypass grafting of the coronary (CABG) or peripheral arteries. Chronic anticoagulant therapy may also be beneficial in preventing the vessel luminal narrowing (restenosis) that often occurs following PTCA and CABG, and in the maintenance of vascular access patency in long-term hemodialysis patients. With respect to the venous vasculature, pathologic thrombus formation frequently occurs in the veins of the lower extremities following abdominal, knee and hip surgery (deep vein thrombosis, DVT). DVT further predisposes the patient to a higher risk of pulmonary thromboembolism. A systemic, disseminated intravascular coagulopathy (DIC) commonly occurs in both vascular systems during septic shock, certain viral infections and cancer. This condition is characterized by a rapid consumption of coagulation factors and their plasma inhibitors resulting in the formation of life-threatening thrombin throughout the microvasculature of several organ systems. The indications discussed above include some, but not all, of the possible clinical situations where anticoagulant therapy is warranted. Those experienced in this field are well aware of the circumstances requiring either acute or chronic prophylactic anticoagulant therapy.

Accumulated experimental evidence has also reflected that prothrombin activation is only one of the biological activities of Factor Xa. EPR-1 (effector cell protease receptor-1, recognizing Factor Xa), is believed to mediate several of the vascular wall interactions by Factor Xa. It has been shown to be expressed on human umbilical vein endothelial cells, rat smooth muscle cells and platelets(CR McKenzie, et al., Arterioscler Thromb Vasc Biol 16 1285-91 (1996); also F Bono, et al., J Cell Physiol 172 36-43 (1997), AC Nicholson, et al., J Biol Chem 271 28407-13 (1996),J.M. Herbert, et al., J Clin Invest 101 993-1000 (1998)). This protease-receptor interaction could mediate not only prothrombinase-catalyzed thrombin generation, but also diverse cellular functions such as cell proliferation, release of PDGF and DNA syntheses. The mitogenic effect of Factor Xa has been reported to be dependent on Factor Xa enzymatic activity (F Bono, et al., J Cell Physiol 172 36-43 (1997), J.M. Herbert, et al., J Clin Invest 101 993-1000 (1998)). TAP for example inhibited the mitogenesis of human and rat cultured vascular smooth muscle cells (F Bono, et al., J Cell Physiol 172 36-43 (1997)). In a study of the rabbit carotid artery air-drying injury model, increased EPR-1 expression is detected after vascular injury. Animals treated with the specific Factor Xa inhibitor, DX-9065a, exhibited less neointimal proliferation. The important regulatory role of Factor Xa in the coagulation process coupled with its mitogenic effects points to Factor Xa's involvement in the formation of thrombin at the luminal surface of the vessel wall and contribution to the atherothrombotic process and abnormal proliferation of vascular cells resulting in restenosis or angiogenesis.

These compounds may be used alone or in combination with other diagnostic, anticoagulant, antiplatelet or fibrinolytic agents. For example adjunctive administration of inhibitors of the activity of Factor Xa with standard heparin, low molecular weight heparin(s), synthetic pentasaccharides, direct thrombin inhibitors (e.g. hirudin, Agratroban (Novastan^{®}, aspirin, fibrinogen receptor antagonists, statins / fibrates streptokinase, urokinase and/or tissue plasminogen activator. The compounds described herein may be administered to treat thrombotic complications in a variety of animals such as primates including humans. Inhibition of factor Xa is useful not only in the anticoagulant therapy of individuals having thrombotic conditions but is useful whenever inhibition of blood coagulation is required such as to prevent coagulation of stored whole blood and to prevent coagulation in other biological samples for testing or storage. Thus, any inhibitor of Factor Xa activity can be added to or contacted with any medium containing or suspected of containing Factor Xa and in which it is desired that blood coagulation be inhibited.

In addition to their use in anticoagulant therapy, Factor Xa inhibitors may find utility in the treatment or prevention of other diseases in which the generation of thrombin has been implicated as playing a physiologic role. For example, thrombin has been proposed to contribute to the morbidity and mortality of such chronic and degenerative diseases as arthritis, cancer, atherosclerosis and Alzheimer's disease by virtue of its ability to regulate many different cell types through specific cleavage and activation of a cell surface thrombin receptor, mitogenic effects, diverse cellular functions such as cell proliferation, for example, abnormal proliferation of vascular cells resulting in restenosis or angiogenesis, release of PDGF and DNA syntheses. Inhibition of Factor Xa will effectively block thrombin generation and therefore neutralize any physiologic effects of thrombin on various cell types.

According to a further feature of the invention there is provided a method for the treatment of a human or animal patient suffering from, or subject to, a physiological condition which can be ameliorated by the administration of an inhibitor of the Factor Xa activity, for example conditions as hereinbefore described, which comprises the administration to the patient of a therapeutically effective amount of the compounds described herein, or a composition containing the compounds described herein. "Effective amount" is meant to describe an amount of compound of the present invention effective in inhibiting the activity of Factor Xa and thus producing the desired therapeutic effect.

The present invention also includes within its scope pharmaceutical formulations which comprise at least one of the compounds described herein in association with a pharmaceutically acceptable carrier.

The pharmaceutical compositions can be administered in a suitable formulation to humans and animals by topical or systemic administration, including oral, inhalational, rectal, nasal, buccal, sublingual, vaginal, parenteral (including subcutaneous, intramuscular, intravenous, intradermal, intrathecal and epidural), intracisternal and intraperitoneal. It will be appreciated that the preferred route may vary with for example the condition of the recipient.

The products according to the invention may be presented in forms permitting administration by the most suitable route and the invention also relates to pharmaceutical compositions containing at least one product according to the invention which are suitabte for use in human or veterinary medicine. These compositions may be prepared according to the customary methods, using one or more pharmaceutically acceptable adjuvants or excipients. The adjuvants comprise, inter alia, diluents, sterile aqueous media and the various non-toxic organic solvents. The compositions may be presented in the form of tablets, pills, granules, powders, aqueous solutions or suspensions, injectable solutions, elixirs or syrups, and can contain one or more agents chosen from the group comprising sweeteners, flavorings, colorings, or stabilizers in order to obtain pharmaceutical acceptable preparations.

The choice of vehicle and the content of active substance in the vehicle are generally determined in accordance with the solubility and chemical properties of the product, the particular mode of administration and the provisions to be observed in pharmaceutical practice. For example, excipients such as lactose, sodium citrate, calcium carbonate, dicalcium phosphate and disintegrating agents such as starch, alginic acids and certain complex silicates combined with lubricants such as magnesium stearate, sodium lauryl sulfate and talc may be used for preparing tablets. To prepare a capsule, it is advantageous to use lactose and high molecular weight polyethylene glycols. When aqueous suspensions are used they can contain emulsifying agents or agents which facilitate suspension. Diluents such as sucrose, ethanol, polyethylene glycol, propylene glycol, glycerol and chloroform or mixtures thereof may also be used.

For parenteral administration, emulsions, suspensions or solutions of the products according to the invention in vegetable oil, for example sesame oil, groundnut oil or olive oil, or aqueous-organic solutions such as water and propylene glycol, injectable organic esters such as ethyl oleate, as well as sterile aqueous solutions of the pharmaceutically acceptable salts, are used. The solutions of the salts of the products according to the invention are especially useful for administration by intramuscular or subcutaneous injection. The aqueous solutions, also comprising solutions of the salts in pure distilled water, may be used for intravenous administration with the proviso that their pH is suitably adjusted, that they are judiciously buffered and rendered isotonic with a sufficient quantity of glucose or sodium chloride and that they are sterilized by heating, irradiation or microfiltration.

Suitable compositions containing the compounds of the invention may be prepared by conventional means. For example, compounds of the invention may be dissolved or suspended in a suitable carrier for use in a nebulizer or a suspension or solution aerosol, or may be absorbed or adsorbed onto a suitable solid carrier for use in a dry powder inhaler.

Solid compositions for rectal administration include suppositories formulated in accordance with known methods and containing at least one compound of formula I or formula II.

Actual dosage levels of active ingredients in the pharmaceutical compositions of this invention may be varied so as to obtain an amount of the active compound(s) that is effective to achieve the desired therapeutic response for a particular patient, composition, and mode of administration. The selected dosage level will depend upon the activity of the particular compound, the route of administration, the severity of the condition being treated, and the condition and prior medical history of the patient being treated. However, it is within the skill of the art to start doses of the compound at levels lower than required for to achieve the desired therapeutic effect and to gradually increase the dosage until the desired effect is achieved. In the adult, the doses are generally from about 0.01 to about 100, preferably about 0.01 to about 10, mg/kg body weight per day by inhalation, from about 0.01 to about 100, preferably 0.1 to 70, more especially 0.5 to 10, mg/kg body weight per day by oral administration, and from about 0.01 to about 50, preferably 0.01 to 10, mg/kg body weight per day by intravenous administration. In each particular case, the doses will be determined in accordance with the factors distinctive to the subject to be treated, such as age, weight, general state of health and other characteristics which can influence the efficacy of the medicinal product.

The products according to the invention may be administered as frequently as necessary in order to obtain the desired therapeutic effect. Some patients may respond rapidly to a higher or lower dose and may find much weaker maintenance doses adequate. For other patients, it may be necessary to have long-term treatments at the rate of 1 to 4 doses per day, in accordance with the physiological requirements of each particular patient. Generally, the active product may be administered orally 1 to 4 times per day. It goes without saying that, for other patients, it will be necessary to prescribe not more than one or two doses per day.

Compounds within the scope of the present invention exhibit marked pharmacological activities according to tests described in the literature which tests results are believed to correlate to pharmacological activity in humans and other mammals. The following pharmacological test results are typical characteristics of compounds of the present invention.

### Enzyme Assays:

The ability of the compounds in the present invention to act as inhibitors of factor Xa, is evaluated by determining the concentration of inhibitor which resulted in a 50% loss in enzyme activity (IC50) using purified enzymes.

All enzyme assays are carried out at room temperature in 96-well microtiter plates using a final enzyme concentration of 1 nM. The concentrations of factor Xa are determined by active site titration and the concentrations of all other enzymes are based on the protein concentration supplied by the manufacturer. Compounds according to the invention are dissolved in DMSO, diluted with their respective buffers and assayed at a maximal final DMSO concentration of 1.25%. Compound dilutions are added to wells containing buffer and enzyme and pre-equilibrated for between 5 and 30 minutes. The enzyme reactions are initiated by the addition of substrate and the color developed from the hydrolysis of the peptide-p-nitroanilide substrates is monitored continuously for 5 minutes at 405 nm on a Vmax microplate reader (Molecular Devices). Under these conditions, less than 10% of the substrate is utilized in all assays. The initial velocities measured are used to calculate the amount of inhibitor which resulted in a 50% reduction of the control velocity (IC50). The apparent Ki values are then determined according to the Cheng-Prusoff equation (IC50 = Ki [1 +[S]/Km]) assuming competitive inhibition kinetics.

An additional in vitro assay may be used to evaluate the potency of compounds according to the invention in normal human plasma. The activated partial thromboplastin time is a plasma-based clotting assay that relies on the in situ generation of factor Xa, its assembly into the prothrombinase complex and the subsequent generation of thrombin and fibrin which ultimately yields the formation of a clot as the assay endpoint. This assay is currently used clinically to monitor the ex vivo effects of the commonly used anticoagulant drug heparin as well as direct acting antithrombin agents undergoing clinical evaluation. Therefore, activity in this in vitro assay is considered as a surrogate marker for in vivo anticoagulant activity.

## Claims

1. A compound selected from the group consisting of
1-(4-Amino-quinazolin-7-ylmethyl)-4-[5-(5-chloro-thiophen-2-yl]-isoxazol-3-ylmethyl]-piperazine-2,3-dione,
(R)-1-(4-Amino-quinazolin-7-ylmethyl)-4-[5-(5-chloro-thiophen-2-yl)-isoxazol-3-ylmethyl]-6-methoxymethyl-piperazin-2-one,
(6R)-1-(4-Amino-quinazolin-7-ylmethyl)-4-[5-(5-chloro-thiophen-2-yl)-isoxazol-3-ylmethyl]-6-ethoxymethyl-piperazin-2-one,
1-(4-Amino-quinazolin-7-ylmethyl)-4-[5-(5-chloro-thiophen-2-yl)-isoxazol-3-ylmethyl]-3-(S)-propyl-piperazin-2-one ditrifluoroacetate,
1-(4-Amino-quinazolin-7-ylmethyl)-4-[5-(5-chloro-thiophen-2-yl)-isoxazol-3-ylmethyl]-3-(S)-methyl-piperazin-2-one ditrifluoroacetate,
1-(4-Amino-quinazolin-7-ylmethyl)-4-[3-(5-chloro-thiophen-2-yl)-isoxazol-5-ylmethyl]-piperazin-2-one ditrifluoroacetate,
1-(4-Amino-quinazolin-7-ylmethyl)-4-[3-(5-chloro-thiophen-2-yl)-isoxazol-5-ylmethyl]-3-methyl-piperazin-2-one ditrifluoroacetate,
1-(4-Amino-quinazolin-7-ylmethyl)-4-[5-(5-chloro-thiophen-2-yl)-isoxazol-3-ylmethyl]-piperazin-2-one ditrifluoroacetate or
4-[5-(5-Chloro-thiophen-2-yl)-isoxazol-3-ylmethyl]-1-(1H-pyrrolo[3,2-c]pyridin-2-ylmethyl)-piperazin-2-one ditrifluoroacetate.

2. A pharmaceutical composition comprising a compound according to claim 1 and a pharmaceutically acceptable carrier.

3. The use of a compound as claimed in claim 1 for the preparation of a medicament for the treatment of acute myocardial infarction, unstable angina, thromboembolism, acute vessel closure associated with thrombolytic therapy, percutaneous transluminal coronary angioplasty (PTCA), transient ischemic attacks, stroke, intermittent claudication, bypass grafting of the coronary or peripheral arteries (CABG), preventing the vessel luminal narrowing (restenosis) that often occur following PTCA and CABG, venous vasculature, pathologic thrombus formation frequently occurring in the veins of the lower extremities following abdominal, knee and hip surgery, deep vein thrombosis or a systemic, disseminated intravascular coagulopathy of several organ systems.

## Patentansprüche

1. Verbindungen ausgewählt aus der Gruppe bestehend aus
1-(4-Aminochinazolin-7-ylmethyl)-4-[5-(5-chlorthiophen-2-yl)isoxazol-3-ylmethyl]piperazin-2,3-dion,
(*R*)-1-(4-Aminochinazolin-7-ylmethyl)-4-[5-(5-chlorthiophen-2-yl)isoxazol-3-ylmethyl]-6-methoxy-methylpiperazin-2-on,
(6*R*)-1-(4-Aminochinazolin-7-ylmethyl)-4-[5-(5-chlorthiophen-2-yl)isoxazol-3-ylmethyl]-6-ethoxymethyl-piperazin-2-on,
1-(4-Aminochinazolin-7-ylmethyl)-4-[5-(5-chlorthiophen-2-yl)isoxazol-3-ylmethyl]-3-(*S*)propylpiperazin-2-on-ditrifluoracetat,
1-(4-Aminochinazolin-7-ylmethyl)-4-[5-(5-chlorthiophen-2-yl)isoxazol-3-ylmethyl]-3-(*S*)methylpiperazin-2-on-ditrifluoracetat,
1-(4-Aminochinazolin-7-ylmethyl)-4-[3-(5-chlorthiophen-2-yl)isoxazol-5-ylmethyl]piperazin-2-on-ditrifluoracetat,
1-(4-Aminochinazolin-7-ylmethyl)-4-[3-(5-chlorthiophen-2-yl)isoxazol-5-ylmethyl]-3-methylpiperazin-2-on-ditrifluoracetat,
1-(4-Aminochinazolin-7-ylmethyl)-4-[5-(5-chlorthiophen-2-yl)isoxazol-3-ylmethyl]piperazin-2-on-ditrifluoracetat oder
4-[5-(5-Chlorthiophen-2-yl)isoxazol-3-ylmethyl]-1-(1H-pyrrolo[3,2-c]pyridin-2-ylmethyl)piperazin-2-on-ditrifluoracetat.

2. Pharmazeutische Zusammensetzung, enthaltend eine Verbindung nach Anspruch 1 und einen pharmazeutisch unbedenklichen Träger.

3. Verwendung einer Verbindung nach Anspruch 1 zur Herstellung eines Medikaments zur Behandlung von akutem Herzinfarkt, instabiler Angina, Thromboembolismus, akutem Gefäßverschluß assoziiert mit thrombolytischer Therapie, perkutaner transluminaler Koronarangioplastie (PTCA), vorübergehenden ischemischen Anfällen, Schlaganfall, Claudicatio intermittens, Legen eines Bypasses bei koronaren oder peripheren Arterien (CABG), Verhindern der Verengung des Gefäßlumens (Restenose), die häufig nach PTCA und CABG auftritt, pathologischer Thrombusbildung in den venösen Gefäßen, die häufig in den Venen der unteren Extremitäten nach Operationen am Bauch, am Knie oder an der Hüfte auftritt, tiefer Venenthrombose oder systemischer disseminierterintravaskulärer Koagulopathie mehrerer Organsysteme.

## Revendications

1. Composé choisi parmi le groupe constitué de :
1-(4-aminoquinazolin-7-ylméthyl)-4-[5-(5-chlorothiophén-2-yl)-isoxazol-3-ylméthyl]-pipérazine-2,3-dione,
(R)-1-(4-aminoquinazolin-7-ylméthyl)-4-[5-(5-chlorothiophén-2-yl)-isoxazol-3-ylméthyl]-6-méthoxyméthyl-pipérazin-2-one,
(6R)-1-(4-aminoquinazolin-7-ylméthyl)-4-[5-(5-chlorothiophén-2-yl)-isoxazol-3-ylméthyl]-6-éthoxy-méthylpipérazin-2-one,
ditrifluoroacétate de 1-(4-aminoquinazolin-7-ylméthyl)-4-[5-(5-chlorothiophén-2-yl)-isoxazol-3-ylméthyl]-3-(S)-propylpipérazin-2-one,
ditrifluoroacétate de 1-(4-aminoquinazolin-7-ylméthyl)-4-[5-(5-chlorothiophén-2-yl)-isoxazol-3-ylméthyl]-3-(S)-méthylpipérazin-2-one,
ditrifluoroacétate de 1-(4-aminoquinazolin-7-ylméthyl)-4-[3-(5-chlorothiophén-2-yl)-isoxazol-5-ylméthyl]-pipérazin-2-one,
ditrifluoroacétate de 1-(4-aminoquinazolin-7-ylméthyl)-4-[3-(5-chlorothiophén-2-yl)-isoxazol-5-ylméthyl]-3-méthylpipérazin-2-one,
ditrifluoroacétate de 1-(4-aminoquinazolin-7-ylméthyl)-4-[5-(5-chlorothiophén-2-yl)-isoxazol-3-ylméthyl]-pipérazin-2-one ou
ditrifluoroacétate de 4-[5-(5-chlorothiophén-2-yl)-isoxazol-3-ylméthyl]-1-(1H-pyrrolo[3,2-c]pyridin-2-ylméthyl)-pipérazin-2-one.

2. Composition pharmaceutique comprenant un composé selon la revendication 1 et un support pharmaceutiquement acceptable.

3. Utilisation d'un composé selon la revendication 1, pour la préparation d'un médicament destiné au traitement de l'infarctus du myocarde aigu, de l'angine instable, du thromboembolisme, de la fermeture d'un vaisseau aiguë associée à une thérapie thrombolytique, de l'angioplastie coronarienne transluminale percutanée (ACTP), des attaques ischémiques transitoires, de l'accident vasculaire cérébral, de la claudication intermittente, du pontage aortocoronarien par greffe (PACG), pour empêcher le rétrécissement luminal de vaisseaux (resténose) qui se produit souvent à la suite d'une ACTP et d'un PACG, du système vasculaire veineux, de la formation de thrombus pathologique qui se produit fréquemment dans les veines des extrémités inférieures à la suite d'une chirurgie abdominale, du genou et de la hanche, de la thrombose veineuse profonde ou d'une coagulopathie intravasculaire disséminée systémique de plusieurs systèmes d'organes.
